## Europäisches Patentamt

(19) **European Patent Office**

**Office européen des brevets**

(11) Publication number: **0 211 267**
**B1**

(12) **EUROPEAN PATENT SPECIFICATION**

(45) Date of publication of patent specification: **05.12.90**

(51) Int. Cl.⁵: **A 61 K 37/02, A 61 K 9/22**

(21) Application number: **86109446.4**

(22) Date of filing: **10.07.86**

(54) **Continuous release peptide compositions.**

(30) Priority: **29.07.85 US 759701**

(43) Date of publication of application:
**25.02.87 Bulletin 87/09**

(45) Publication of the grant of the patent:
**05.12.90 Bulletin 90/49**

(84) Designated Contracting States:
**AT BE CH DE FR GB IT LI LU NL SE**

(56) References cited:
**EP-A-0 013 949**
**EP-A-0 133 988**
**EP-A-0 158 277**
**US-A-2 517 513**
**US-A-4 411 890**

(73) Proprietor: **AMERICAN CYANAMID COMPANY**
**1937 West Main Street P.O. Box 60**
**Stamford Connecticut 06904-0060 (US)**

(72) Inventor: **Cady, Susan Mancini**
**1501 Revere Road**
**Yardley Pennsylvania 19067 (US)**
Inventor: **Fishbein, Richard**
**18 Meadow Run Road**
**Skillman New Jersey 08558 (US)**

(74) Representative: **Wächtershäuser, Günter, Dr.**
**Tal 29**
**D-8000 München 2 (DE)**

Courier Press, Leamington Spa, England.

**Description**

The difficulties encountered in the development of methods and compositions which continuously release pharmaceutical preparations in a uniform manner over extended periods of time are well known.

Recent developments in the area of controlling the release of drugs have been disclosed which describe methods for controlling the release of drugs by microencapsulation and containment within a biodegradable matrix.

The present invention provides a novel composition for the parenteral administration of an essentially uniform and continuous amount of a peptide over an extended period of time comprising a compacted and partially coated fatty acid salt of a peptide, which exhibits some degree of solubility in an aqueous physiological environment. The peptide for use in this composition is

$$his\text{-}D\text{-}trp\text{-}ala\text{-}trp\text{-}D\text{-}phe\text{-}lys\text{-}NH_2.$$

Surprisingly, it has been found that for the partially coated implant of compacted $C_{10}$—$C_{20}$ fatty acid salts of the peptide

$$his\text{-}D\text{-}trp\text{-}ala\text{-}trp\text{-}D\text{-}phe\text{-}lys\text{-}NH_2$$

that in general, the dissolution rate decreases with increasing carbon content of the fatty acid used to prepare the salt, while this is not the case when the salts are uncoated.

The invention also is directed to the preparation and administration of said compositions.

Fig. 1 is a graph of the dissolution of uncoated, compacted $C_{10}$—$C_{20}$ fatty acid peptide salts.

Fig. 2 is a graph of the dissolution of compacted and partially coated $C_{10}$—$C_{20}$ fatty acid peptide salts.

Fig. 3 is a graph showing the effect of different coatings on the dissolution profile of the tristearate salt of the peptide.

The rate of release and dosage of the composition of the invention may be adjusted by the use of different fatty acid salts and coating materials. A preferred group of fatty acid salts for use in this invention include the $C_{10}$—$C_{20}$ fatty acid salts of the peptide

$$his\text{-}D\text{-}trp\text{-}ala\text{-}trp\text{-}D\text{-}phe\text{-}lys\text{-}NH_2.$$

An advantage of the composition of the invention is that once the dissolution characteristics of the compacted and partially coated fatty acid salt of the peptide to be administered has been determined, the salt may be assayed, which provides for exact control of dosages, as the pure compacted salt is the implant. A further advantage of the composition of the invention is the linear nature of its dissolution profile.

The composition of the invention may optionally contain up to about 50% by weight of a diluent or mixture of diluents and up to about 5% by weight of a lubricant or mixture of lubricants. Suitable diluents for the invention are ethyl cellulose and castorwax. A suitable lubricant for the invention is magnesium stearate.

The term "aqueous physiological environment" means the body of a warm-blooded animal as well as such an *in vitro* environment which may be mimicked by aqueous liquids, such as phosphate buffered solutions at a temperature of 35°C to 40°C.

An implant for the administration of peptide may be prepared by admixing the peptide

$$his\text{-}D\text{-}trp\text{-}ala\text{-}trp\text{-}D\text{-}phe\text{-}lys\text{-}NH_2$$

in an organic solvent such as methanol with a sufficient amount of the desired fatty acid to provide for complete salt formation. The salt may then be isolated by removing the solvent, washing and drying. The isolated pure salt is pressed by compaction or extrusion into an implant, preferably a cylindrical implant. The compacted material is coated with a biodegradable or non-biodegradable coating by conventional techniques. Prior to implantation a specified area of the coating is removed to expose the compacted salt. For example, the coating may be cut off at one or both of the ends of a cylindrical implant.

It has been shown in an *in vivo* steer study that the desirable dissolution characteristics of the compositions of the invention are maintained and that the release profiles for *in vivo* release and *in vitro* dissolution are comparable, demonstrating the effectiveness of the present compositions for the parenteral administration of a fatty acid salt of a peptide. In a controlled experiment, an implant of the tristearate salt of the peptide described above, released an average of 1.8% of the peptide present in the implant *in vivo* per day compared to 1.6% per day *in vitro*.

The invention is further illustrated by the following examples.

Example 1
Preparation of the tristearate salt of $his\text{-}D\text{-}trp\text{-}ala\text{-}trp\text{-}D\text{-}phe\text{-}lys\text{-}NH_2$

Stearic acid (1.025 g, 3.6 mmol) is added to a solution of the peptide

his-D-trp-ala-trp-D-phe-lys-NH$_2$

(1.0 g, 0.9 mmol) in methanol (15 mL) and the resulting solution is heated in a warm water bath for three hours. Evaporation of the methanol under reduced pressure produces a white powder product which is washed with ether (2440 mL), filtered and dried to yield 0.509 g, 74.7% of the desired tristearate salt with an elemental analysis C, 67.13; H, 9.23; N, 9.83%. Calculated analysis for C$_{99}$H$_{165}$N$_{12}$O$_{15}$; C, 67.43; H, 9.43; N, 9.53%.

Examples 2—10

Utilizing the procedure of Example 1 and substituting the appropriate C$_{10}$—C$_{20}$ fatty acid yields the tri-fatty C$_{10}$—C$_{20}$ fatty acid salts of the peptide

his-D-trp-ala-trp-D-phe-lys-NH$_2$

listed in Table I below.

TABLE I

| Fatty acid | Fatty acid | | Peptide | | Product | | Molecular formula | Molecular weight | Elemental analysis | | | | | |
|---|---|---|---|---|---|---|---|---|---|---|---|---|---|---|
| | | | | | | | | | Found | | | Calculated | | |
| | GMS | mM | GMS | MM | GMS | % Yield | | | % C | % H | % N | % C | % H | % N |
| C10 | 0.31 | 1.8 | 0.5 | 0.458 | 0.65 | 80.7 | $C_{75}H_{117}N_{12}O_{15}$ | 1426.782 | 63.29 | 8.17 | 11.60 | 63.13 | 8.27 | 11.78 |
| C11 | 0.503 | 2.7 | 0.75 | 0.675 | 0.74 | 75.0 | $C_{78}H_{123}N_{12}O_{15}$ | 1468.86 | 63.59 | 8.35 | 11.57 | 63.78 | 8.44 | 11.44 |
| C12 | 0.72 | 3.6 | 1.0 | 0.9 | 0.9 | 67.4 | $C_{81}H_{129}N_{12}O_{15}$ | 1509.912 | 64.68 | 8.42 | 11.34 | 64.46 | 8.62 | 11.14 |
| C13 | 0.386 | 1.8 | 0.5 | 0.458 | 0.567 | 80.0 | $C_{84}H_{135}N_{12}O_{15}$ | 1551.99 | — | — | — | — | — | — |
| C14 | 0.82 | 3.6 | 1.0 | 0.9 | 0.86 | 60.0 | $C_{87}H_{141}N_{12}O_{15}$ | 1595.094 | 65.03 | 8.65 | 10.67 | 65.51 | 8.91 | 10.54 |
| C15 | 0.87 | 3.6 | 1.0 | 0.9 | 0.88 | 58.8 | $C_{90}H_{147}N_{12}O_{15}$ | 1637.172 | 65.99 | 8.74 | 10.55 | 66.02 | 9.05 | 10.27 |
| C16 | 0.92 | 3.6 | 1.0 | 0.9 | 0.86 | 57.0 | $C_{93}H_{153}N_{12}O_{15}$ | 1679.25 | 66.49 | 8.95 | 10.37 | 66.51 | 9.18 | 10.01 |
| C17 | 0.97 | 3.6 | 1.0 | 0.9 | 0.9 | 59.0 | $C_{96}H_{159}N_{12}O_{15}$ | 1721.328 | — | — | — | — | — | — |
| C20 | 1.717 | 5.49 | 1.5 | 1.37 | 1.65 | 63.2 | $C_{105}H_{177}N_{12}O_{15}$ | 1847.562 | 67.97 | 9.57 | 9.23 | 68.25 | 9.66 | 9.098 |

Examples 11—20

Preparation of the partially coated compacted implants of tri $C_{10}$—$C_{20}$ fatty acid salts of the peptide

Implants are prepared by weighing a sufficient quantity of the desired $C_{10}$—$C_{20}$ tri-fatty acid salt of the peptide

$$his\text{-}D\text{-}trp\text{-}ala\text{-}trp\text{-}D\text{-}phe\text{-}lys\text{-}NH_2$$

to provide approximately 200 mg of the peptide as the triacetate equivalent. The salt is then compressed on a carver press at from 70 to 350 bar (1000 to 5000 psig) in a 4.8 mm (3/16″) diameter cylindrical die. Smaller implants (providing ~53 mg of peptide triacetate equivalents) are prepared by compressing the appropriate quantity of tri $C_{10}$—$C_{20}$ fatty acid salt on a rotary tablet press using a 3.2 mm (1/8″) diameter punch and die to give cylindrical implants.

The implants prepared above are coated with both biodegradable and non-biodegradable coatings by procedures A and B below.

Procedure A

Non-biodegradable silicon polymer

Clean grade silicon elastomer (10 parts) is mixed with curing agent (one part) on a watch glass with a spatula. This is deaerated in a dessicator for 30 minutes. The implants are grasped by the ends with tweezers, rolled into the silicon polymer, placed on end on aluminum foil and cured at 40°C for five hours. One or both of the ends are removed with a razor blade leaving the "shaft" of the cylinder coated.

Alternatively, implants may be dip coated with 20% to 40% of a medical adhesive, sold under the trademark SILASTIC® by Dow Corning, which has been dispersed in hexane, and dried and cured at 40°C to 50°C overnight before removing the coating from one or both of the base ends.

Procedure B

Biodegradable coatings

The polymer or copolymer (one part) is dissolved in chloroform (three to eight parts). Each implant is grasped by the ends with tweezers, dipped into the polymer solution, and then the chloroform evaporated at room temperature. Each implant is coated twice. After the coating dried overnight at room temperature, the polymer ends are removed with a razor blade, leaving the long cylindrical "shaft" coated.

Tables II and III below contain a summary of the physical data on the coated implants thus prepared.

## TABLE II

| Salt | mg Peptide triacetate equivalents | Coating | | | | | Implant length (mm) |
|------|-----------------------------------|---------|--------|----------|------|----------|---------------------|
| | | None | pGLA* | Silastic** | mg | mm Thick | |
| C10 | 197.55 | X | — | — | — | — | 12.305 |
| | 197.7 | — | X | — | 37.3 | 0.38 | 12.455 |
| | 194.09 | — | — | X | 53.1 | 0.485 | 12.265 |
| C11 | 198.61 | X | — | — | — | — | 12.325 |
| | 196.74 | — | X | — | 33.9 | 0.36 | 12.31 |
| | 195.75 | — | — | X | 41.2 | 0.42 | 13.09 |
| C12 | 193.7 | X | — | — | — | — | 12.11 |
| | 196.46 | — | X | — | 36.1 | 0.3755 | 12.03 |
| | 173.98 | — | — | X | 37.8 | 0.44 | 11.05 |
| C13 | 199.8 | X | — | — | — | — | 13.4 |
| | 166.4 | — | X | — | 17.9 | 0.045 | 11.285 |
| | 168.9 | — | — | X | 62.0 | 0.55 | 11.475 |
| C14 | 179.71 | X | — | — | — | — | 12.4 |
| | 198.9 | — | X | — | 19.8 | 0.0775 | 13.23 |
| | 197.2 | — | — | X | 70.0 | 0.5325 | 13.21 |
| C15 | 190.0 | X | — | — | — | — | 14.405 |
| | 184.0 | — | X | — | 20.7 | 0.045 | 13.45 |
| | 189.0 | — | — | X | 68.7 | 0.585 | 13.365 |
| C16 | 196.3 | X | — | — | — | — | 14.455 |
| | 197.6 | — | X | — | 30.8 | 0.155 | 14.18 |
| | 203.9 | — | — | X | 82.5 | 0.4525 | 15.05 |
| C17 | 167.7 | X | — | — | — | — | 13.23 |
| | 161.6 | — | X | — | 25.8 | 0.37 | 13.465 |
| | 192.1 | — | — | X | 89.2 | 0.355 | 14.035 |
| C18 | 176.59 | X | — | — | — | — | 13.31 |
| | 177.29 | — | X | — | 42.9 | 0.3975 | 13.41 |
| | 181.09 | — | — | X | 89.4 | 0.55 | 14.11 |

*Copoly(L-lactide/glycolide) ηinh 0.32, 55/45 L-lactide/glycolide
**Silicon polymer nonbiodegradable

## TABLE III

| Salt | mg Peptide triacetate equivalents | Coating | | | | | Implant length (mm) |
|------|------|------|------|------|------|------|------|
| | | None | pGLA* | Silastic** | mg | mm Thick | |
| C13 | 52.74 | X | — | — | — | — | 8.225 |
| | 47.96 | — | X | — | 7.8 | 0.5 | 8.39 |
| | 48.52 | — | — | X | 24.1 | .065 | 8.145 |
| C15 | 50.98 | X | — | — | — | — | 9.0 |
| | 50.26 | — | X | — | 6.3 | 0.04 | 10.0 |
| | 49.6 | — | — | X | 16.3 | 0.06 | 9.25 |
| C18 | 50.28 | X | — | — | — | — | 8.33 |
| | 51.65 | — | X | — | 3.4 | 0.0475 | 8.495 |
| | 51.46 | — | — | X | 13.7 | 0.1225 | 9.0 |
| C20 | 50.59 | X | — | — | — | — | 10.15 |
| | 49.18 | — | X | — | 4.8 | 0.05 | 9.37 |
| | 51.36 | — | — | X | 15.7 | 0.07 | 10.0 |

*Copoly(L-lactide/glycolide), 55/45 L-lactide/glycolide, $\eta$inh 0.48, S14384-52A.

Example 21

Dissolution experiments

Dissolution experiments are carried out at 39°C using a shaking bottle method. Phosphate buffered saline (PBS) adjusted to pH—7.1 is the dissolution medium ($NaH_2PO_4 \cdot H_2O$ 3.45 g, $Na_2HPO_4$ 3.55 g NaCl 9.50 g dissolved in distilled water to 1000 mL).

The implant is placed in a disposable polypropylene flat base tube (Sarstedt No. 58.537 with No. 67.790 cap) and 30 mL PBS is added. The tubes are placed in a 39°C Fisher Shaking Water Bath (Model 129—shaker setting at $2\frac{3}{4}$). The PBS is changed daily and samples are assayed by optical density measurement at 210 nm to give the dissolution profiles.

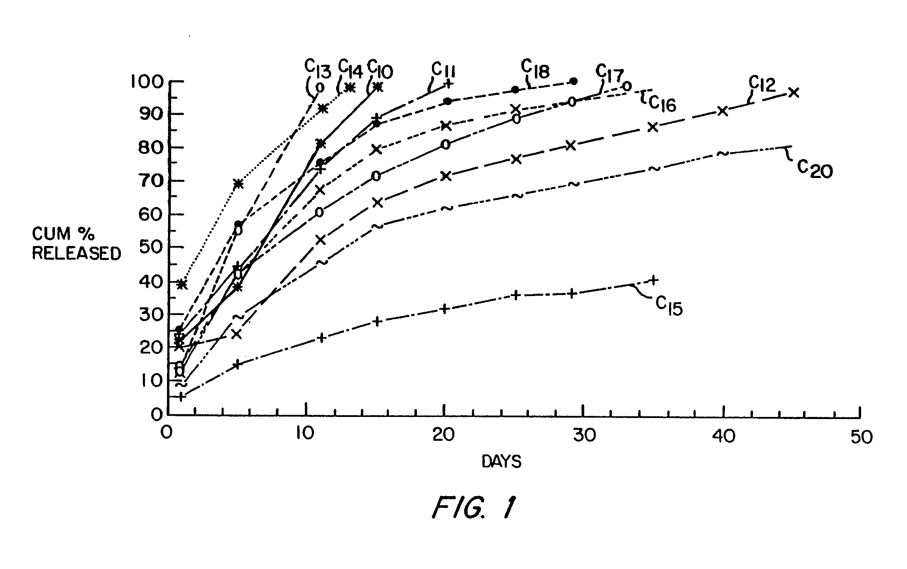

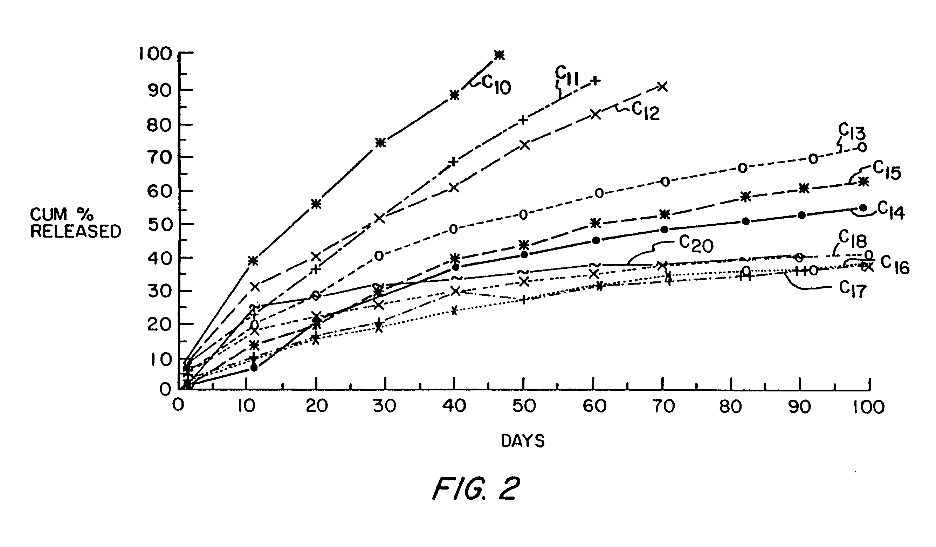

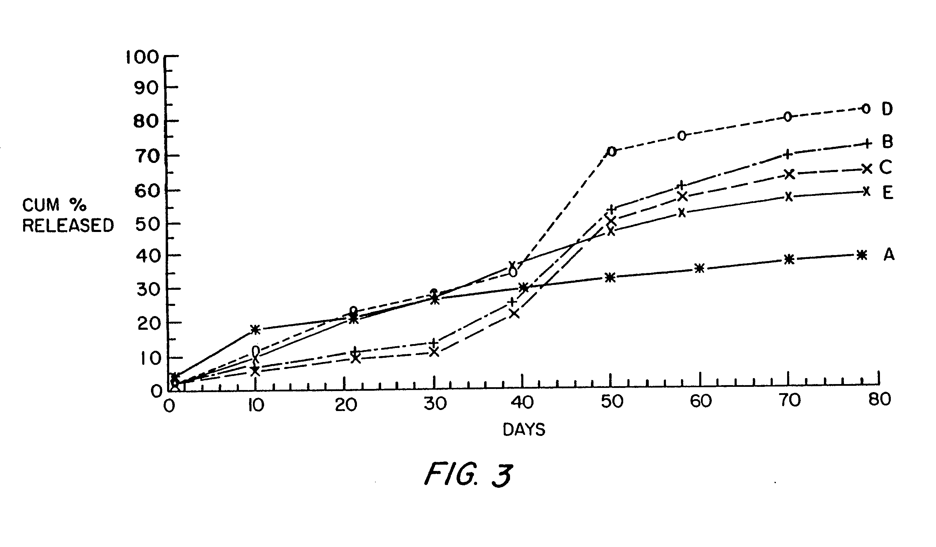

The results of these experiments are summarized in Figs. 1, 2, and 3 below. Fig. 1 shows the dissolution of uncoated, compacted implants of $C_{10}$—$C_{20}$ fatty acid salts of the peptide

his-D-trp-ala-trp-D-phe-lys-NH$_2$

in an aqueous physiological environment at 39°C from 10 to 48 days. The dissolution kenetics and rate are altered by coating the cylindrical implant and having one or both of the ends exposed. Fig. 2 shows that complete dissolution for many of the implants has not occurred after 100 days. The use of different $C_{10}$—$C_{20}$ fatty acid salts in conjunction with various coatings on the compacted implants provides a means of regulating the release rate of the peptide over extended periods of time. Fig. 3 represents the variations in the release profiles obtained with the tristearate salt of the peptide

his-D-trp-ala-trp-D-phe-lys-NH$_2$

obtained with some different biodegradable and nonbiodegradable coating materials. In Fig. 3, A is a nonbiodegradable silicon polymer; B is a biodegradable (L-lactide/glycolide, 57/43); C is a biodegradable (L-lactide/glycolide, 71.6/28.4); biodegradable (poly-L-lactide) and E is biodegradable (tetramethylene carbonate/glycolide 50/50).

Example 22

Dissolution characteristics of the partially coated compacted implants of the tristearate salt of his-D-trp-ala-trp-D-phe-lys-NH$_2$ when administered to a steer (in vivo)

I. Preparation of H$^3$ labeled peptide tristearate salt

H$^3$ labeled peptide triacetate (17.2 mg H$^3$, 0.24 mCi/mg, 0.0158 mM; and 413 mg unlabeled, 0.378 mM) are dissolved in distilled water (45 mL) in a centrifuge tube (Falcon No 2070—plastic). Ammonium hydroxide (30% NH$_3$) is added dropwise until basic (pH—10). White particles immediately precipitated. After ten minutes, the tube is put into a beaker of ice water and left for 20 minutes. The tubes are centrifuged for 15 minutes (~2600 rpm, Damon—IEC Clinical Centrifuge Model CL with Model No. 801 rotor). The water is decanted into another centrifuge tube and three drops ammonium hydroxide is added. After five minutes the tube is put into a beaker of ice water and left for 20 minutes. The three tubes are centrifuged as above. The water layer is decanted into a 473 ml (pint) plastic bottle for freezing, storage and recycling. Each tube of solid centrifuge material is dissolved in methanol (15 mL) and put into one 200 mL recovery flask. Stearic acid (1.32 g, 4.72 mM) is added and the flask is put into a 600 mL beaker of warm water. This methanol solution is stirred until dissolved and then for another three hours. The product is concentrated *in vacuo* to give a white solid. This is washed with ether (2×40 mL), filtered, dried in a dessicator over drierite overnight yielding 662.3 mg (4.49 mCi).

## II. Implant fabrication

A Carver Press (Model M, 25 ton press) is used for implant fabrication. The $H^3$ labeled salts for the dissolution implants are cut with cold tristearate (1:3 hot to cold) and thoroughly mixed with a mortar and pestle before compression while the animal implants are prepared directly from $H^3$ salts. Two tristearate salt implants (one for dissolution, one for animal implantation) are compressed at 350 bar (5,000 psi) in a custom made 4.8 mm (3/16″) die to give cylinders.

## III. Implant coating

A biodegradable polymer coating is applied by dissolving poly(glycolic acid/lactic acid) copolymer (1 g, 71.6/28.4 L-lactide/glycolide ratio, ηinh 0.51) in chloroform (3 mL). Each of the implants is grasped by the ends with tweezers and dipped into the polymer solution, then the chloroform is evaporated off at room temperature in a hood. Each implant is coated twice, and dried overnight at room temperature, the polymer ends are removed with a razor blade, leaving the long cylindrical "shaft" coated.

## IV. Peptide implant administration and behavior *in vivo*

Under local anesthetic, a small slit is made at the base of the ear on a 340.2 kg (750 lb) steer. A pocket is made subcutaneously and the $H^3$ labeled tristearate salt implant is inserted with forceps. The slit is sutured to prevent the implant from coming out.

The steer is maintained in a cattle metabolism cage. It is given up to 4.54 kg (ten lb) daily of Cattle Growing Ration No. 632. Hay and water are provided *ad libitum*. The total amount of urine and feces are collected daily, volume and weight are measured and representative samples are sent for radioactivity determination. Blood samples are taken periodically to assay peptide blood levels.

Calculations of *in vivo* peptide release are based on the daily total amount of radioactivity appearing in the urine and feces.

## V. Comparative *in vitro* dissolution

Dissolution experiments are carried out at 39°C using a shaking bottle method. Phosphate buffered saline (PBS) adjusted to pH=7.1 is the dissolution medium ($NaH_2PO_4 \cdot H_2O$ 3.45 g, $Na_2HPO_4$ 3.55 g, NaCl 9.50 g is dissolved in distilled water to 1000 mL).

The implant is placed in a disposable polypropylene flat base tube (Sarstedt No. 58.537 with No. 65.790 cap) and 30 mL PBS is added. The tubes are placed in a 39°C Fisher Shaking Water with (Model 129-shaker setting at $2\frac{3}{4}$).

The PBS is changed daily and samples are assayed for peptide released by counting using the scintillation counter. During the course of the experiment some of the samples had to be diluted 1 to 10 with PBS before counting.

## VI. Results and discussion

Table VI summarizes the steer labeled tristearate data *in vitro* and *in vivo*. In the *in vitro* release profile, the implant released 1.6—2.5 mg/day after the initial burst until the pGLA (copoly-L-lactide/glycolide) coating started to lose integrity after three weeks. The *in vivo* release system, calculated from the daily excretion of radiolabeled material, is similar to the *in vitro* profile. The *in vivo* implant released an average of 3.2 mg/day over the 43 day experiment. The peptide is found at an increasing level in the steer's blood over time.

TABLE VI
Steer/tristearate data

| | In vitro | | In vivo | | | | Daily waste | | |
|---|---|---|---|---|---|---|---|---|---|
| Day | mg/day | Cum % | Urine mg/day | Feces mg/day | Total mg/day | Total cum % | mL urine | kg feces | Blood level mg/steer |
| 1 | 3.70 | 1.9 | 0.43 | 4.50 | 4.93 | 2.8 | 1800.0 | 3.0 | 0.154 |
| 2 | 2.10 | 3.0 | 0.53 | 6.80 | 7.33 | 5.9 | 3460.0 | 7.0 | 0.159 |
| 3 | 2.01 | 4.0 | 0.35 | 2.40 | 2.75 | 7.6 | 3310.0 | 9.9 | 0.146 |
| 4 | 2.70 | 5.4 | 0.28 | 1.98 | 2.26 | 8.8 | 3250.0 | 11.9 | — |
| 5 | 1.65 | 6.2 | 0.25 | 2.30 | 2.55 | 10.2 | 3260.0 | 9.8 | — |

TABLE VI (Cont'd)

| Day | In vitro | | In vivo | | | | Daily waste | | Blood level mg/steer |
|---|---|---|---|---|---|---|---|---|---|
| | mg/day | Cum % | Urine mg/day | Feces mg/day | Total mg/day | Total cum % | mL Urine | kg feces | |
| 6 | 1.65 | 7.1 | 0.28 | 1.47 | 1.75 | 11.2 | 3920.0 | 7.4 | 0.163 |
| 7 | 1.60 | 7.9 | 0.27 | 1.57 | 1.84 | 12.3 | 4020.0 | 9.7 | 0.41 |
| 8 | 1.60 | 8.7 | 0.30 | 2.24 | 2.54 | 13.7 | 3000.0 | 8.8 | — |
| 9 | 1.55 | 9.5 | 0.32 | 1.78 | 2.10 | 14.9 | 3050.0 | 5.0 | — |
| 10 | 1.68 | 10.4 | 0.35 | 2.44 | 2.79 | 16.5 | 2400.0 | 5.2 | 0.305 |
| 11 | 1.71 | 11.3 | 0.32 | 1.72 | 2.04 | 17.7 | 2650.0 | 11.0 | — |
| 12 | 1.71 | 12.1 | 0.24 | 1.92 | 2.16 | 19.0 | 2640.0 | 8.9 | — |
| 13 | 1.71 | 13.0 | 0.31 | 2.29 | 2.60 | 20.5 | 3510.0 | 9.2 | 0.279 |
| 14 | 2.26 | 14.2 | 0.26 | 1.58 | 1.84 | 21.6 | 3090.0 | 7.2 | — |
| 15 | 1.56 | 15.0 | 0.30 | 1.84 | 2.14 | 22.8 | 2930.0 | 8.0 | 0.349 |
| 16 | 2.60 | 16.3 | 0.32 | 1.65 | 1.97 | 23.9 | 3340.0 | 8.9 | — |
| 17 | 2.14 | 17.4 | 0.43 | 3.45 | 3.88 | 26.1 | 4000.0 | 11.4 | 0.289 |
| 18 | 2.16 | 18.5 | 0.39 | 2.57 | 3.00 | 27.8 | 4040.0 | 7.4 | — |
| 19 | 2.16 | 19.6 | 0.52 | 4.12 | 4.64 | 30.5 | 4340.0 | 7.4 | — |
| 20 | 2.16 | 20.7 | 0.48 | 2.57 | 3.05 | 33.7 | 3540.0 | 5.4 | 0.388 |
| 21 | 5.57 | 23.6 | 0.59 | 4.16 | 4.75 | 36.4 | 4540.0 | 9.6 | — |
| 22 | 5.90 | 26.6 | 0.52 | 2.11 | 2.63 | 37.9 | 4040.0 | 4.2 | 0.471 |
| 23 | 5.87 | 29.6 | 0.67 | 3.21 | 3.88 | 40.2 | 2980.0 | 5.6 | — |
| 24 | 6.01 | 32.7 | 0.62 | 4.17 | 4.80 | 43.0 | 2620.0 | 10.8 | 0.513 |
| 25 | 5.16 | 35.4 | 0.52 | 6.12 | 6.64 | 46.8 | 2929.0 | 8.8 | — |
| 26 | 5.16 | 38.0 | 0.54 | 4.33 | 4.87 | 49.6 | 3400.0 | 10.5 | — |
| 27 | 5.16 | 40.7 | 0.72 | 4.23 | 4.95 | 52.4 | 3440.0 | 9.8 | 0.584 |
| 28 | 6.28 | 43.9 | 0.90 | 8.20 | 9.10 | 57.6 | 4140.0 | 8.6 | — |
| 29 | 5.31 | 46.6 | 0.67 | 5.57 | 6.24 | 61.2 | 3560.0 | 5.6 | — |
| 30 | 4.16 | 48.8 | 0.55 | 3.37 | 3.90 | 63.5 | 3040.0 | 7.9 | — |
| 31 | 4.22 | 50.9 | 0.45 | 4.05 | 4.50 | 66.1 | 3540.0 | 12.8 | 0.790 |
| 32 | 3.20 | 52.6 | 0.38 | 3.56 | 3.94 | 68.3 | 4740.0 | 9.2 | — |
| 33 | 3.20 | 54.2 | 0.36 | 3.49 | 3.85 | 70.5 | 4500.0 | 9.3 | — |
| 34 | 2.90 | 55.7 | 0.36 | 2.52 | 2.88 | 72.2 | 4320.0 | 3.8 | — |

TABLE VI (Cont'd)

| | In vitro | | In vivo | | | | Daily waste | | |
|---|---|---|---|---|---|---|---|---|---|
| Day | mg/day | Cum % | Urine mg/day | Feces mg/day | Total mg/day | Total cum % | mL Urine | kg Feces | Blood level mg/steer |
| 35 | 2.90 | 57.2 | 0.35 | 2.28 | 2.63 | 73.7 | 3660.0 | 6.2 | — |
| 36 | 3.80 | 59.2 | 0.31 | 1.61 | 1.92 | 75.2 | 3000.0 | 6.2 | 0.800 |
| 37 | 3.74 | 61.1 | 0.22 | 1.46 | 1.68 | 76.1 | 2920.0 | 6.3 | — |
| 38 | 3.86 | 63.0 | 0.17 | 0.40 | 0.57 | 76.5 | 1040.0 | 3.4 | 0.700 |
| 39 | 2.01 | 64.1 | 0.84 | 0.90 | 1.74 | 77.5 | 3640.0 | 5.1 | — |
| 40 | 2.01 | 65.1 | 0.09 | 0.85 | 0.94 | 78.0 | 1360.0 | 8.8 | — |
| 41 | 2.01 | 66.1 | 0.14 | 0.54 | 0.68 | 78.4 | 3440.0 | 9.2 | 0.720 |
| 42 | 3.06 | 67.7 | 0.09 | 0.10 | 0.19 | 78.5 | 2480.0 | 6.5 | — |
| 43 | 2.70 | 69.1 | 0.04 | 0.18 | 0.22 | 78.6 | 440.0 | 2.3 | 0.740 |
| | 1.6 per day | | | | | 1.8 per day | | | |

## Claims

1. A composition for the parenteral administration of an essentially uniform and continuous amount of a peptide over an extended period of time which comprises a compacted and partially coated fatty acid salt of the peptide, which exhibits some degree of solubility in an aqueous physiological environment wherein the coating is biodegradable or non-biodegradable, the fatty acid salt is selected from a $C_{10}$—$C_{20}$ fatty acid, and the peptide is

his-D-trp-ala-trp-D-phe-lys-$NH_2$.

2. A composition according to Claim 1, wherein the salt is tristearate.

3. A method for administering parenterally an essentially uniform and continuous amount of a peptide over an extended period of time which comprises implanting a compacted and partially coated fatty acid salt of the peptide, which exhibits some degree of solubility in an aqueous physiological environment, wherein the coating is biodegradable or non-biodegradable, the fatty acid is selected from a $C_{10}$—$C_{20}$ fatty acid, and the peptide is

his-D-trp-ala-trp-D-phe-lys-$NH_2$.

4. A method according to Claim 3, wherein the salt is the tristearate.

5. A method for preparing an implant for the parenteral administration of an essentially uniform and continuous amount of a peptide over an extended period of time which comprises compacting a fatty acid salt of the peptide, which exhibits some degree of solubility in an aqueous physiological environment, into the form of an implant; coating the implant with a coating material; and removing a section of the coating material from the coated implant to expose the peptide, wherein the coating is biodegradable or non-biodegradable, the fatty acid is selected from a $C_{10}$—$C_{20}$ fatty acid, and the peptide is

his-D-trp-ala-trp-D-phe-lys-$NH_2$.

6. A method according to Claim 5, wherein the salt is tristearate.

## Patentansprüche

1. Mittel für die parenterale Verabreichung einer im wesentlichen einförmigen und kontinuierlichen Menge eines Peptids über einen längeren Zeitraum, umfassend ein kompaktiertes und teilweise beschichtetes Fettsäuresalz des Peptids, das in einer wässrigen und physiologischen Umgebung ein gewisses Maß an Löslichkeit zeigt, wobei die Beschichtung bioabbaubar oder nicht-bioabbaubar ist, das Fettsäuresalz ausgewählt ist aus einer $C_{10}$—$C_{20}$-Fettsäure und das Peptid his-D-trp-ala-trp-D-phe-lys-NH$_2$

ist.

2. Mittel gemäß Anspruch 1, wobei das Salz Tristearat ist.

3. Verfahren zur parenteralen Verabreichung einer im wesentlichen einförmigen und kontinuierlichen Menge eines Peptids über einen längeren Zeitraum, umfassend das Implantieren eines kompaktierten und teilweise beschichteten Fettsäuresalzes des Peptids, das in einer wässrigen, physiologischen Umgebung ein gewisses Maß an Löslichkeit zeigt, wobei die Beschichtung bioabbaubar oder nicht-bioabbaubar ist, die Fettsäure ausgewählt ist aus einer C$_{10}$—C$_{20}$-Fettsäure, und das Peptid his-D-trp-ala-trp-D-phe-lys-NH$_2$

ist.

4. Verfahren gemäß Anspruch 3, wobei das Salz das Tristearat ist.

5. Verfahren zur Herstellung eines Implantats für die parenterale Verabreichung einer im wesentlichen einförmigen und kontinuierlichen Menge eines Peptids über einen längeren Zeitraum, umfassend die Kompaktierung eines Fettsäuresalzes des Peptids, das in einer wässrigen, physiologischen Umgebung ein gewisses Maß an Löslichkeit zeigt, in die Form eines Implantats, Beschichtung des Implantats mit einem Beschichtungsmaterial und Entfernung eines Abschnitts des Beschichtungsmaterials von dem beschichteten Implantat, um das Peptid freizulegen, wobei die Beschichtung bioabbaubar oder nicht-bioabbaubar ist, die Fettsäure ausgewählt ist aus einer C$_{10}$—C$_{20}$-Fettsäure und das Peptid his-D-trp-ala-trp-D-phe-lys-NH$_2$

ist.

6. Verfahren gemäß Anspruch 5, wobei das Salz-Tristearat ist.

**Revendications**

1. Composition pour l'administration à peu près régulière et continue, par voie parentérale, d'une quantité d'un peptide pendant une période prolongée, comprenant un sel d'acide gras du peptide, compacté partiellement enrobé, ayant un certain degré de solubilité dans un milieu physiologique aqueux; dans laquelle l'enrobage est biodégradable ou non biodégradable, l'acide gras salifié est choisi parmi un acide gras en C$_{10}$ à C$_{20}$, et le peptide est:

his-D-trp-ala-trp-D-phe-lys-NH$_2$

2. Composition selon la revendication 1, dans laquelle le sel est le tristéarate.

3. Procédé d'administration à peu près régulière et continue, par voie parentérale, d'une quantité d'un peptide pendant une période prolongée, selon lequel on implante un sel d'acide gras du peptide, compacté et partiellement enrobé, ayant un certain degré de solubilité dans un milieu physiologique aqueux, l'enrobage étant biodégradable ou non biodégradable, l'acide gras étant choisi parmi les acides gras en C$_{10}$ à C$_{20}$, et le peptide étant:

his-D-trp-ala-trp-D-phe-lys-NH$_2$

4. Procédé selon la revendication 3, dans lequel le sel est le tristéarate.

5. Procédé de préparation d'un implant pour l'administration à peu près régulière et continue, par voie parentérale, d'une quantité d'un peptide pendant une période prolongée, selon lequel on compacte sous la forme d'un implant, un sel d'acide gras du peptide, ayant un certain degré de solubilité dans un milieu physiologique aqueux; on revêt l'implant avec un matériau d'enrobage; et on élimine une partie du matériau d'enrobage de l'implant enrobé, pour exposer le peptide, l'enrobage étant biodégradable ou non biodégradable, l'acide gras étant choisi parmi les acides gras en C$_{10}$ à C$_{20}$, et le peptide étant:

his-D-trp-ala-trp-D-phe-lys-NH$_2$

6. Procédé selon la revendication 5, dans lequel le sel est le tristéarate.

FIG. 1

FIG. 2

FIG. 3